# EUROPEAN PATENT APPLICATION

(11) **EP 3 961 649 A1**
(43) Date of publication of application: **02.03.2022**
(21) Application number: 20193923.8
(22) Date of filing: 01.09.2020
(51) Int. Cl.: G16H 50/30, G16H 50/70

(54) **DISPLAYING A RISK SCORE**

(71) Applicant: Koninklijke Philips N.V., 5656 AG Eindhoven (NL)
(72) Inventor: OP DEN BUIJS, Jorn, 5656 AE Eindhoven (NL); PIJL, Marten Jeroen, 5656 AE Eindhoven (NL); PAUWS, Steffen Clarence, 5656 AE Eindhoven (NL); MEULENDIJKS, Lydia, 5656 AE Eindhoven (NL)
(74) Representative: Philips Intellectual Property & Standards

(57) **Abstract**

According to an aspect, there is provided a computer implemented method of displaying to a user a risk score associated with a risk of a patient requiring a medical intervention. The method comprises obtaining the risk score for the patient; determining a format in which to display the risk score to the user based on a numerical literacy of the user; and sending an instruction to a user display to instruct the user display to display the risk score to the user in the determined format.

## Description

### FIELD OF THE INVENTION

The disclosure herein relates to displaying to a user a risk score associated with a risk of a patient requiring a medical intervention.

### BACKGROUND OF THE INVENTION

Personal Emergency Response Systems (PERS), can help the elderly maintain independence and freedom at home. A PERS system may comprise a wearable device, such as a necklace or wristband that is worn by a patient (e.g. elderly person) and contains an emergency button. When the patient is in need of help, they can press the button to get in contact with a response center. This way, a PERS provides the patient with immediate contact to a user of the PERS system (such as a trained response agent/telehealth worker) that assesses - together with the patient - what type of help is needed. Depending on the severity of the case, actions can vary from calling a relative to alerting emergency medical services. Interactions between a PERS patient and a call center may be documented by the response agent using structured and unstructured data entries in an electronic record. An example PERS device is the Philips "Lifeline" device.

A predictive analytics engine may use the data collected by a PERS service to generate a predictive risk score of the likelihood that the patient requires an emergency hospital transport within the next 30 days. The risk score can be presented on a dashboard to a user such as a medical professional or case manager, who can contact the more high-risk patients, based on the risk scores, to further assess their health and - if deemed necessary - schedule an intervention with the aim of avoiding emergency hospital admissions. This can save on health care costs in the long run and can help elderly patients to live independently at home for longer. An example predictive analytics engine is the Philips "CareSage" product.

Telehealth systems (such as Philips Telehealth) provide care delivery to chronically ill persons/patients outside of the hospital. In this home care delivery, a clinical back-office at a healthcare facility (with nurses) or a call center of trained call agents can monitor patients on their health status and wellbeing, triage and escalate certain patients for intervention. The monitoring can take place using predictive risk scores that describe a risk of the patient worsening and/or needing a medical intervention. Such risk score then need to be interpreted by back-office or call center representatives.

Continuous Positive Airway Pressure (CPAP) is a common treatment of patients with obstructive sleep apnea. For a good therapeutic response, patients need to adhere to the therapy by using the CPAP device over-night for a pre-specified number of hours for an extended period. Many patients struggle to comply with the therapy due to inconvenience and configuration of the device and its peripherals. An adherence score predicting the possibility that the patient will not achieve the pre-specified level of adherence is another example where a risk score can be sent to a care provider to initiate further assistance or guidance.

The disclosure herein relates to the processing of such risk scores presented in healthcare systems, such as these, and other systems where risk scores are provided.

### SUMMARY OF THE INVENTION

As described above, various telehealth and health monitoring services use predicted risk scores e.g. describing the risk that a patient will require intervention. The success/utility of these predicted risk analyses however depends in part on the communication of predictive risk scores to the medical professional / case manager / nurse. It is desirable that the case manager has a good comprehension of the risk score to treat the patient with the right care and the right "urgency".

The comprehension of the risk score is asymmetrically influenced by many factors, including the level of the baseline population-average risk and the predicted risk-level. For example, if the population average risk is low, say 3%, and the predicted risk is six times higher at 18%, then presenting the predicted risk as an absolute percentage increase (+15%) may be perceived differently than presenting the predicted risk as a risk ratio (6 times increased risk), as a relative risk increase (+600%) or as natural frequencies (1 out of 18). If the baseline risk is higher, say 10%, then a 15% absolute risk increase would amount to an estimated risk of 25%, a risk ratio of 2.5 times and a relative risk increase of +250%. These two simple, but real world examples already demonstrate the confusion in the interpretation of risks.

Risk information can be framed according to how it is presented and can drive people (including medical experts) into particular direction of decision and action.

It is an objective of embodiments herein to improve on the presentation of risk scores to a user (e.g. in a telehealth context), so as to improve understanding and standardise responses.

Thus, according to a first aspect, there is provided a computer implemented method of displaying to a user a risk score associated with a risk of a patient requiring a medical intervention. The method comprises obtaining the risk score for the patient; determining a format in which to display the risk score to the user based on a numerical literacy of the user; and sending an instruction to a user display to instruct the user display to display the risk score to the user in the determined format.

Thus, the format or manner in which the risk score is displayed to the user is selected based on their comprehension of different possible formats. In this way, a format that is most likely to be accurately comprehended by the user is presented to them (e.g. in a personalised manner) so as to enable the user to make an appropriate decision. In some embodiments herein, the numerical literacy, (e.g. the user's understanding or interpretation of risk scores presented in different format types) may also be used to influence the user to perform actions in accordance with a system goal (e.g. to reduce costs, or reduce false alarms etc).

According to a second aspect there is an apparatus for displaying to a user a risk score associated with a risk of a patient requiring a medical intervention. The apparatus comprises a memory comprising instruction data representing a set of instructions and a processor configured to communicate with the memory and to execute the set of instructions. The set of instructions, when executed by the processor, cause the processor to: obtain the risk score for the patient; determine a format in which to display the risk score to the user based on a numerical literacy of the user; and send an instruction to a user display to instruct the user display to display the risk score to the user in the determined format.

According to a third aspect there is a computer program product comprising a computer readable medium, the computer readable medium having computer readable code embodied therein, the computer readable code being configured such that, on execution by a suitable computer or processor, the computer or processor is caused to perform the method of the first aspect.

These and other aspects will be apparent from and elucidated with reference to the embodiment(s) described hereinafter.

### BRIEF DESCRIPTION OF THE DRAWINGS

Example embodiments will now be described, by way of example only, with reference to the following drawings, in which:
Fig. 1 illustrates an apparatus according to some embodiments herein;
Fig. 2 illustrates a system according to some embodiments herein;
Fig. 3 illustrates a method according to some embodiments herein; and
Fig. 4 illustrates a process according to some embodiments herein.

### DETAILED DESCRIPTION OF EMBODIMENTS

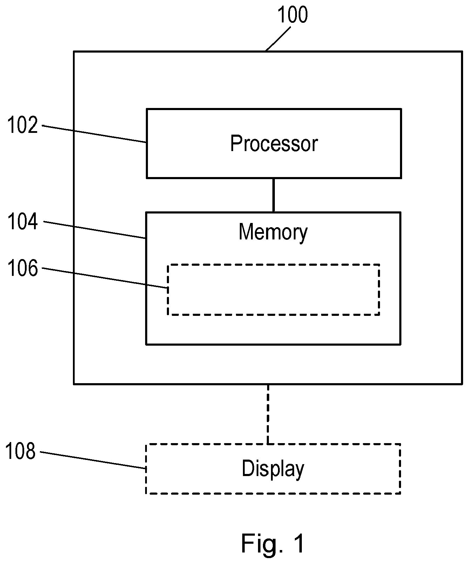

Turning now to Fig. 1 in some embodiments there is an apparatus 100 for displaying to a user a risk score associated with a risk of a patient requiring a medical intervention, according to some embodiments herein. Generally, the apparatus may form part of a computer apparatus or system e.g. such as a laptop, desktop computer or other computing device. In some embodiments, the apparatus 100 may form part of a distributed computing arrangement or the cloud. The apparatus 100 may form part of a PERS system, a telehealth system, or a CPAP monitoring system, as described above. It will be appreciated however that these are merely examples and that embodiments of the apparatus 100 may be comprised in other systems for monitoring patients (or subscribers of the system) where risk scores are presented to users of the system. Further examples include but are not limited to, a COVID-19 risk assessment system, and a system for predicting whether a patient will suffer from side effects of a treatment (e.g. such as a treatment for cancer).

The apparatus comprises a memory 104 comprising instruction data 106 representing a set of instructions and a processor 102 (e.g. processing circuitry or logic) configured to communicate with the memory and to execute the set of instructions 106. Generally, the set of instructions, when executed by the processor, may cause the processor to perform any of the embodiments of the method 300 as described below.

Embodiments of the apparatus 100 may be for use in displaying to a user a risk score associated with a risk of a patient requiring a medical intervention. More specifically, the set of instructions 106, when executed by the processor 102, cause the processor 102 to: obtain the risk score for the patient, determine a format in which to display the risk score to the user based on a numerical literacy of the user, and send an instruction to a user display 108 to instruct the user display to display the risk score to the user in the determined format.

The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store instruction data 106 (e.g. program code) that can be executed by the processor 102 to perform the method described herein. Alternatively or in addition, one or more memories 104 may be external to (i.e. separate to or remote from) the apparatus 100. For example, one or more memories 104 may be part of another device. Memory 104 can be used to store the risk score, the format types and/or any other information or data received, calculated or determined by the processor 102 of the apparatus 100 or from any interfaces, memories or devices that are external to the apparatus 100. The processor 102 may be configured to control the memory 104 to store the risk score, the format types and/or any other information or data received, calculated or determined by the processor 102.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus 100 may further comprise, or be in communication with a display 108. A display 108 may comprise, for example, a computer screen, and/or a screen on a mobile phone or tablet. The apparatus may further comprise a user input device, such as a keyboard, mouse or other input device that enables a user to interact with the apparatus, for example, to provide initial input parameters to be used in the method 300 described herein. The apparatus 100 may comprise a battery or other power supply for powering the apparatus 100 or means for connecting the apparatus 100 to a mains power supply.

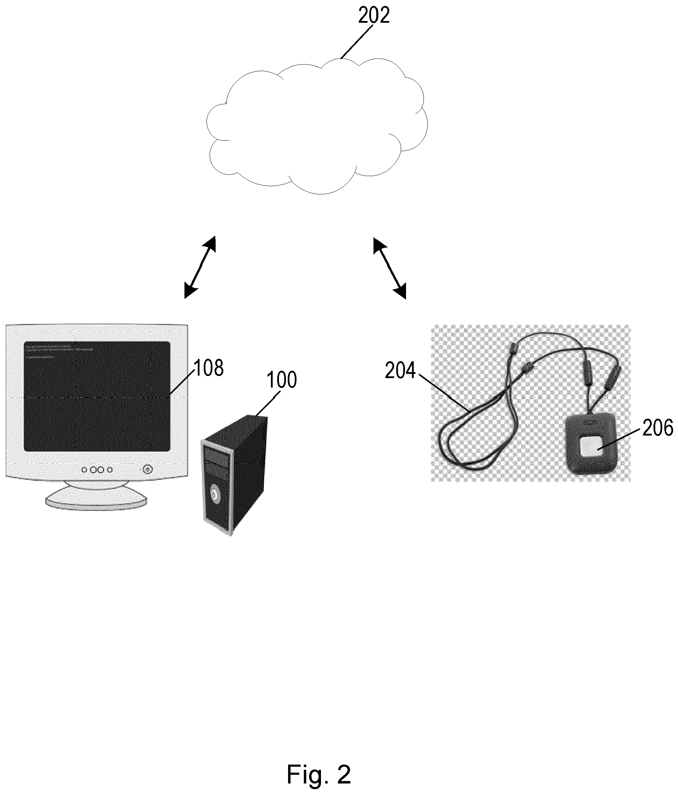

Turning to Fig. 2 the apparatus 100 may form part of a system. For example, such as a PERS system, or a telehealth system. In an example of a PERS system, a patient (e.g. elderly or vulnerable person) may wear a monitor 204 comprising an emergency button 206. The monitor and PERS system comprising the apparatus 100 described above may be in communication via the internet 202.

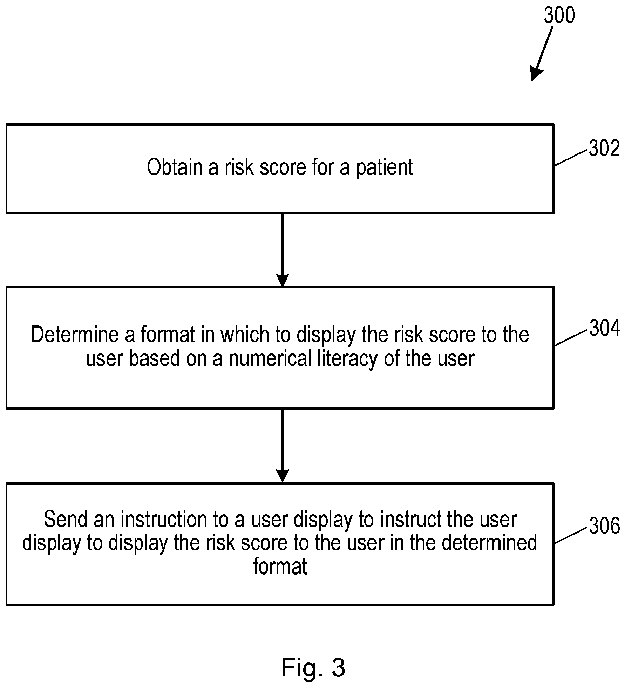

Turning to Fig. 3, there is a computer implemented method 300 for use in displaying to a user a risk score associated with a risk of a patient requiring a medical intervention. Embodiments of the method 300 may be performed, for example by an apparatus such as the apparatus 100 described above (e.g. including but not limited to apparatuses such as a PERS system, a telehealth system, or a CPAP monitoring system as described above).

Briefly, in a first step 302, the method 300 comprises: obtaining the risk score for the patient. In a second step 304, the method comprises determining a format in which to display the risk score to the user based on a numerical literacy of the user. In a third step 306, the method comprises sending an instruction to a user display to instruct the user display to display the risk score to the user in the determined format.

As noted above, the preferences and numeracy of the user can play a role in the interpretation of predicted risk scores. Uncertainty about the meaning of numerical information, resulting from lower numeracy, may promote affective interpretations of information about risks (i.e., fearful interpretations) and about benefits (i.e., hopeful interpretations). Selecting a format in which to display the risk score according to the numerical literacy of the user may thus enable a format to be chosen that offers the greatest possibility that the user will accurately comprehend the risk and/or action it appropriately. In some embodiments herein, the user's numerical literacy or understanding/interpretation of different format types may also be used to influence them to perform actions in accordance with a system goal (e.g. to reduce cost, reduce false alarms etc).

In more detail, as used herein, the user may be a medical professional/expert or clinician, a carer, a relative of the patient, or a telehealth operative such as a call centre agent or case manager. The patient may comprise any individual who is registered with the system, such as an elderly or vulnerable person or a patient registered with a doctor's surgery, hospital or other physician. In other words, a patient may be a subscriber of the system.

In step 302, the method comprises obtaining a risk score for the patient. The risk score may comprise a risk or probability associated with a risk of a patient requiring a medical intervention. For example, a risk that a patient will experience an adverse event that requires (medical) intervention, e.g. to prevent the event from happening. It may comprise a risk that a patient will need an intervention within a given time frame, for example, within the next 30 days. It will be appreciated that these are merely examples and that the risk score may represent other types of risk to those described herein, for example, the risk that a patient may have a fall, the risk that a patient may have a heart attack, a risk associated with contracting an illness such as, for example, a COVID-19 risk score, a hospital re-admission risk score, a risk score associated with the patient having side effects (e.g. of cancer treatment), or any other risk or probability score.

Examples of interventions include, for example, hospital admission for the patient, initiating a house visit to check on the patient, and an appointment being made with a health professional. In some embodiments, an intervention may comprise measures to be taken at a regional level to protect high-risk or vulnerable people, or to help the healthcare system to cope with health challenges (e.g. COVID-19).

The risk score may be determined based on historical data, or from sensor data acquired from the patient or patient's home. The risk score may thus comprise an estimation or prediction that an event will occur requiring intervention. The risk score may be output by one or more models. The risk score may be determined, for example, by a statistical model, a model trained using a machine learning process, or any other model that can be used to predict a risk score.

The step 302 of obtaining a risk score may further comprise processing or converting the obtained score. For example, the mean risk score from a reference cohort may further be obtained and a risk score may be processed with reference to the mean risk score from the reference cohort.

In some embodiments, the risk score may be determined (e.g. calculated) by the system 100. In other embodiments the risk score may be obtained (e.g. requested) from a remote server or other computing device.

In step 304, the method comprises determining a format in which to display the risk score to the user, based on a numerical literacy of the user. In some embodiments, for example, the format may be selected from a list of possible formats.

As used herein a format may comprise any type of format that may be used to convey information to the user. A format may comprise, for example, a numerical format e.g. the risk score may be presented as a percentage, fraction, a percentage risk compared to a baseline (population) risk, a comparison to a mean reference risk score, an absolute risk, a natural frequency, odds, odds ratio, hazard ratio, likelihood ratio, etc.

A format may comprise a text format e.g. describing that the patient is at a "High" or "Low" risk. These may be described as verbal quantifications e.g. 'high', 'moderate' or 'low', or other verbal terms to explain the probability of an event happening like 'common' or 'rare'. It is known that verbal descriptors often result in overestimation of actual risks; people might have different quantification for these verbal terms. For example, the paper by Sanne J.W. Willems, Casper J. Albers and Ionica Smeets (2019) entitled "Variability in the interpretation of Dutch probability phrases - a risk for miscommunication" shows that different people interpret verbal cues (such as "likely", "some chance" and "maybe") very differently when asked to express them on a numerical scale.

Another method is to communicate the risk score using visualizations; standard bar charts, pie charts or icon arrays are ways to visualize risks. A format may thus comprise a graphical format e.g. a plot of risk over time. In other examples, the format may comprise an auditory format, for example, the system may make a sound to indicate that the person is at high risk. In another example, a format may comprise a tactile format, for example, a user device associated with the apparatus 100 may vibrate when a person is at high risk.

As noted above, in step 304 of the method 300, a format is determined that is personalised to the user, based on the numerical literacy of the user. As used herein, the numerical literacy of the user may comprise any indication of how the user comprehends, or perceives risk scores when presented in different formats. This may be based on the user's ability to use and understand mathematics. It could also be based on the user's perception of urgency associated with scores presented in different formats, or how the user interacts with the system when presented with risk scores in different formats.

The user's numerical literacy may be assessed. For example, using a questionnaire or test. An example of such a questionnaire is provided in Annex 1, and an example test is provided in Annex 2. It will be appreciated that these are merely examples however, and that the numerical literacy of a user may be assessed in a variety of ways. Results of such a questionnaire may be used, for example, to calculate an aggregate form of the numeracy (e.g., weighted average). In embodiments described below that use machine learning models, the results of such a questionnaire may be assessed by a designer of the system and labelled with a ground truth label of the most appropriate format for the user (or ground truth labels of the circumstances in which different labels might be used).

In some embodiments the user may, for example, be asked to rank risk scores displayed using different formats. In some embodiments, the user may be asked to convert a risk score presented in one format to another format (e.g. to determine how the user comprehends risk scores presented in different formats). For example, verbal probability descriptors could be quantified with a question such as: *"Please give your point estimate of your numerical interpretation as a percentage (or a scale of 1 to 100) of the percentage likelihood of an event occurring if the event is described as: i) impossible, ii) never, iii) very unlikely, iv) almost impossible, v) almost never, vi) rarely, vii) unlikely, viii) low change, ix) not often, x) sometime, xi) common, xii) uncommon, and xiii) rare."*

In some embodiments, the step 304 may comprise selecting a format that standardises a user's interpretation of the risk score, e.g. compared to a cohort of other users. For example, if a first user converts a risk of "sometimes" as 60% and "common" as 70%, but a second user rates "sometimes" as 70%, then, the first user may be presented with "common" in the same circumstances as the second user is presented with "sometimes".

In some embodiments, the step 304 may comprise determining a format that is most likely to be understood by the user, based on the numerical literacy of the user. For example, if the user has poor numerical literacy, then verbal quantifiers may be used "high", "low" instead of "80%" or "20%". As another example, if the user understands fractions better than percentages, then it may be determined to present the risk score to the user as a fraction.

In some embodiments, the step 304 may further comprise determining a cost effectiveness of performing the medical intervention. The step of determining a format in which to display the risk score to the user may be further based on the determined cost effectiveness. For example, the step of determining a format may comprise selecting a format that is more likely to result in the user initiating the medical intervention if the medical intervention is determined to be cost effective compared to if the medical intervention is determined to be less cost effective. In other embodiments, determining a format may comprise selecting a format that is more likely to result in the user initiating the medical intervention if a cost associated with not performing the medical intervention is higher than a cost associated with performing the medical intervention. In other words, if an intervention is determined to be cost effective, then based on the user's numerical literacy (e.g. understanding or interpretation of numerical formats), a format may be selected that increases the likelihood that the user will act on the risk score. The user's understanding/interpretation of different formats may thus be used to select a format that will encourage the user to perform an action in response to the risk score that is cost effective. E.g. a format may be chosen for the user that will be interpreted by the user as being more urgent.

Furthermore, patient characteristics and history may play a role in the risk score communication. As an example, one might want to reduce the urgency of the predicted risk for a patient, if there were previously many false alarms. One way to do this might be by presenting it as an absolute percentage rather than a risk ratio. Vice versa, for patients with COPD or congestive heart failure, higher urgency might be desirable if the risk outcome is on hospital admission due to the high associated costs and patient burden of a potential hospital admission.

Thus in some embodiments, the step of determining a format comprises selecting a format that is less likely to result in the user initiating the medical intervention if previous risk scores displayed to the user have resulted in the user initiating unnecessary medical interventions, compared to if previous risk scores displayed to the user have resulted in the user initiating necessary medical interventions.

In some embodiments, the step 304 may comprise the use of decision rules to determine which numerical format to display. For example one or more decision rules (if-then-else statements) may be used to determine the appropriate format for displaying the risk score. As noted above, "appropriate" can be defined as: leading to the highest case manager comprehension, leading to a standardised interpretation compared to a cohort, leading to the most cost-effective intervention strategy, influencing a case manager interpretation of over- or underestimating actual risk, or any other consideration that may be desirable to take into account and influence the user based on. An example set of decision-based rules are given in Annex 3.

In other embodiments, the user's numerical literacy and / or the appropriate format can be estimated based on the case manager's interaction with the apparatus (e.g. the dashboard of the PERS or telehealth system). Metrics for this may include how fast the case manager acts based on different risk score formats, the speed of comprehension of other numerical aspects in the dashboard, or by linking patient outcomes to the case manager's estimation of risk (i.e., the accuracy of the case manager's risk estimations). The system can then learn and adapt the risk score representation based on these metrics.

In some embodiments the step of determining a format in which to display the risk score to the medical professional user may comprise using a model trained using a machine learning process to predict the format in which to display the risk score to the user, based on one or more input parameters related to the numerical literacy of the user. For example, the model may be been trained using training data comprising training examples, each training example comprising: example values of the one or more input parameters related to a numerical literacy of an example user and a ground truth format (e.g. clinical outcome or cost of care) for said user.

For example, the ground truth format may comprise a format that would lead the example user to correctly determine whether to initiate an example medical intervention. As noted above, in some embodiments, the ground truth format may comprise a format that would lead the example user to interpret the risk score in a standardised manner (e.g. compared to a cohort of other users). A ground truth may be assigned for each example user, for example, by the architect of the system, who may determine the appropriate format for each user based, for example, on their response to a questionnaire or test (e.g. as shown in Annexes 1 and 2).

In some embodiments, the ground truth may comprise a clinical outcome, such as an emergency department visit and/or a cost of such medical care. In such examples, the machine learning model may learn to output the format (given the numerical literacy of the user and other input parameters) that would lead to the user initiating interventions that result in improved clinical outcomes (e.g., a reduction in ED visits) and/or lower cost of care, as compared to a reference population.

The skilled person will be familiar with machine learning models that can be trained to provide (e.g. predict) an appropriate output e.g. such as a classification, based on a set of input parameters. For example, in some embodiments, the machine learning model may comprise a neural network. In one example, a numeral network may be configured to take as input, parameters related to the numerical literacy of a user and output a format for said user. In another example, the neural network may be trained to output the risk score in the determined format for the user (e.g. ready for display). In another example, a neural network further comprise providing feedback to the reinforcement model. The feeplurality of possible formats, a likelihood that said format will lead the user to make the most optimal decision. The format(s) with the highest likelihood may then be presented to the user.

The skilled person will be familiar with neural networks, but in brief, neural networks are a type of supervised machine learning model that can be trained to predict a desired output for given input data. Neural networks are trained by providing training data comprising example input data and the corresponding "correct" or ground truth outcome that is desired. Neural networks comprise a plurality of layers of neurons, each neuron representing a mathematical operation that is applied to the input data. The output of each layer in the neural network is fed into the next layer to produce an output. For each piece of training data, weights associated with the neurons are adjusted until the optimal weightings are found that produce predictions for the training examples that reflect the corresponding ground truths. A neural network may be trained in this manner, using method such as back-propagation and gradient descent.

In other embodiments, the model may comprise a reinforcement learning model. The skilled person will be familiar with reinforcement learning and reinforcement learning agents, however, briefly, reinforcement learning is a type of machine learning process whereby a reinforcement learning agent (e.g. algorithm) is used to perform actions on a "system" to adjust the "system" according to an objective (which may, for example, comprise moving the system towards an optimal or preferred state of the system). The reinforcement learning agent receives a reward based on whether the action changes the system in compliance with the objective (e.g. towards the preferred state), or against the objective (e.g. further away from the preferred state). The reinforcement learning agent therefore performs actions (e.g. makes recommendations) with the goal of maximising the rewards received.

Put more formally, a reinforcement learning agent receives an observation from the environment in state S and selects an action to maximize the expected future reward r. Based on the expected future rewards, a value function V for each state can be calculated and an optimal policy π that maximizes the long term value function can be derived.

In the context of this disclosure, the PERS, or telehealth system is the "environment" in the state S. The state S may include, the health or status of the patients, the cost associated with running the system etc. The "observations" are the effects of presenting a user with a risk score in a particular format and the "actions" performed by the reinforcement learning agents are the recommendations made by the reinforcement learning agent of which format to display the risk scores to the users. Generally, the reinforcement learning agents herein may receive feedback in the form of a reward or credit assignment every time they recommend a format in which to display a risk score to a user. As noted above, the goal of the reinforcement learning agents herein may be to e.g. minimise cost, minimise hospital admissions, or optimise a cost/number of hospital admissions metric. The feedback received may depend on whether displaying a risk score in the format recommended by the reinforcement learning agent encouraged the user to action the risk score in a way consistent with, or contrary to the goal(s).

Thus in some embodiments, the method 300 may further comprise providing feedback to the reinforcement model. The feedback may indicate, for example, whether the user correctly initiated the medical procedure when the risk score was displayed in the determined format (e.g. as recommended by the reinforcement learning agent).

Thus, in summary, reinforcement learning may be used, e.g. in the context of the "multi-armed bandit" model that learns how to optimize a policy of when to use what numeric risk score format in which context to lead to optimal decisions, or achieve a particular predefined state. In other words a reinforcement learning model could be used to determine which risk formats should be used for each user in each circumstance in order to minimise hospital admissions, minimise costs, and/or optimise the number of hospital admissions for a given cost. The use of reinforcement learning models has the advantage that the reinforcement learning model may adapt over time in a self-learning manner. For example, the system may adapt according to the achieved results, namely the comprehension of the risk score by the medical expert and/or the cost effectiveness of preventive measures to avoid hospitalization. The goals of a reinforcement learning model may also be easily adapted (e.g. by changing a reward scheme), if the priorities of the system need to be changed.

In another embodiment, the model may use a combination of logic rules and neural network approach, such as a Fuzzy Neural Network, or differential Inductive Logic Programming. In some embodiments, certain rules and/or relationships may be predetermined. E.g., for a user initiating unnecessary interventions (e.g. with more false positives) it may be desirable to display a risk score in a format that the user perceives as being less urgent. Or a risk score for a patient with heart failure may be presented using a format that the user will perceive as more urgent. These rules can be either set in stone (logic rules) or their relationships defined (Fuzzy). The relationships with the other input parameters still may be less clear and can be represented with a neural network. In such cases a combination of a black box neural network with logic and/or fuzzy rules may be used so as to incorporate such rules (or guidelines) into the neural network framework.

Turning to step 306, the method then comprises sending an instruction to a user display to instruct the user display to display the risk score to the user in the determined or chosen format.

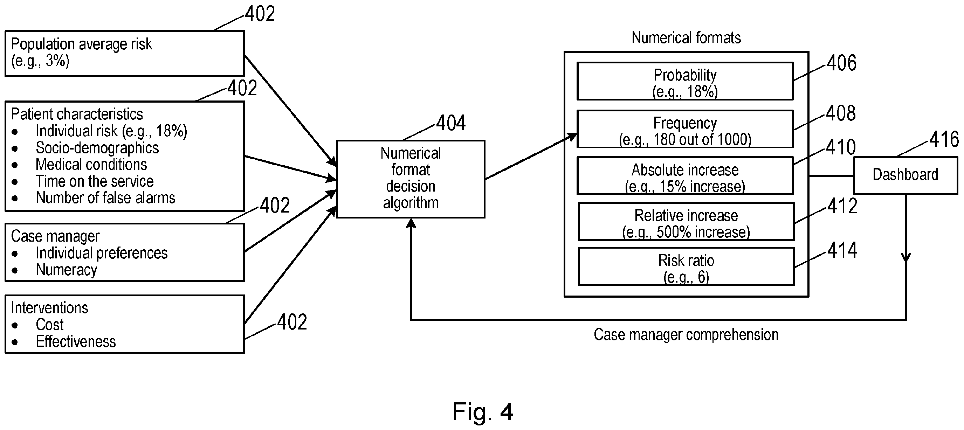

Fig. 4 illustrates a system according to an embodiment herein. In this embodiment, step 302 comprises obtaining a risk score for the patient and other input parameters 402, and providing the input parameters to a numerical format decision algorithm 404.

In this embodiment, the numerical format decision algorithm 404 may take input data from various sources. For example, it may take as input a population average risk. This can be derived from an aggregated historical database, or obtained from medical literature. From this, one obtains a base rate (or prevalence) of events occurring in the common population or target cohort. In another embodiment, one can arrive at conditional base rates presenting the likelihood for high and low risk group within the population or cohort. The intent of the base rate is to evaluate or compare risk scores of individuals by the medical expert.

It may take input from a database with individual patient characteristics, such as the individual predicted risk, as well as other features ranging from socio-demographics and medical conditions, to number of prior false alarms upon an intervention call. It may further take input from a database with preferences and numeracy of the user. It may further take as input quantification measurements of verbal descriptors by the case manager. Information about the cost and effectiveness of interventions (potentially on patient-specific level or on medical condition level) may also be provided as input.

The numerical format decision algorithm may perform the step 304 of the method 300 (according to any of the embodiments described above with respect to the method 300) and determine a format for the user from a predefined list of formats (e.g. formats 406 to 414 in Fig. 4).

The selected format is then displayed on a dashboard 416 to the user. The resulting actual decision or action of the user (as an overt outcome) may be recorded. The outcome may be evaluated in relation to the task at hand, costs and benefits to the patient/subscriber and/or the healthcare organization.

Feedback on the actions taken by the user in response to the risk score being displayed in the recommended format may then be fed back to the numerical format decision algorithm for further training. In this way there is provided a self-learning system that selects a format for the user of the system in order to use the numerical literacy (e.g. understanding of different formats) in order to guide the user to make decisions that further a goal of the system.

In another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### Annex 1

**Table 1: Example questionnaire for obtaining a user's subjective numerical literacy**

| | |
|---|---|
| **Cognitive abilities** | How good are you at working with fractions? |
| | How good are you at working with percentages? |
| | How good are you at calculating a 15% tip? |
| | How good are you at figuring out how much a shirt will cost of it is 25% off? |
| **Preference for display numeric information** | When reading the newspaper, how helpful do you find tables and graphs that are part of the story? |
| | 1 = not at all, 6 = extremely |
| | When people tell you the chance of something happening, do you prefer that they use worlds ("it rarely happens") or numbers ("there's a 1% chance")? |
| | 1 = always prefer words, 6 = always prefer numbers When you hear a weather forecast, do you prefer predictions using percentages (e.g. "there will be a 20% chance of rain today") or predications using only words (e.g. "there is a small chance of rain today")? |
| | 1 = always prefer percentages, 6 = always prefer words (reverse coded) How often do you find numerical information to be useful? |
| | 1 = never, 6 = very often |

### Annex 2

**Table 2: Example questionnaire to evaluate a user's comprehension of a risk score**

| | |
|---|---|
| **Interpretation** | What is the chance that this person will be taken to a hospital within the next 30 days? |
| | .. out of 100 |
| | How many times bigger/smaller than the average person is the risk of this person to be taken to the hospital within the next 30 days? |
| | .. Times ... |
| **Risk perception** | How likely do you think this person will be brought to a hospital within the next thirty days? |
| | Not at all likely - very likely |
| | Do you expect this person to be brought to a hospital in the next thirty days? |
| | Not at all - very much |
| **Affective perception** | How worried are you about the risk of this person? |
| | Very worried - not at all worried |
| | How frightened are you about the risk of this person? |
| | Very frightened - not at all frightened |
| | How severe do you think the risk of this person is? |
| | Very severe - not at all severe |
| | How comfortable are you with the risk of this person? |
| | Very comfortable - not at all comfortable |
| **Decision making** | How likely you think it is that you would call this person? (imagining yourself as the case manager) |
| | Not at all likely - very likely |
| | I as a case manager should call this person based on his/her Risk Score |
| | Totally agree - totally disagree |

### Annex 3

**Table 3: Example decision rules that can be used by the invention to display the risk score in a certain numerical format**

| **Numerical format decision rules** |
|---|
| IF (intervention cost = low) AND (intervention effectiveness = high) AND (patient risk = high) THEN (numerical format = risk ratio) |
| IF (medical condition = heart failure) AND (patient risk = high) THEN (numerical format = risk ratio) |
| IF (patient false alarm rate = high) AND (patient risk = high) THEN (numerical format = absolute percentage) |

## Claims

1. A computer implemented method of displaying to a user a risk score associated with a risk of a patient requiring a medical intervention, the method comprising:
obtaining the risk score for the patient;
determining a format in which to display the risk score to the user based on a numerical literacy of the user; and
sending an instruction to a user display to instruct the user display to display the risk score to the user in the determined format.

2. A method as in claim 1 wherein the format comprises a numerical format, a graphical format or a text format.

3. A method as in claim 1 or 2 wherein the step of determining a format in which to display the risk score to the user comprises determining a format that is most likely to be understood by the user, based on the numerical literacy of the user.

4. A method as in claim 1, 2, or 3 wherein the step of determining a format in which to display the risk score to the user comprises:
using a model trained using a machine learning process to predict the format in which to display the risk score to the user, based on one or more input parameters related to the numerical literacy of the user.

5. A method as in claim 4 wherein the model has been trained using training data comprising training examples, each training example comprising: example values of the one or more input parameters related to a numerical literacy of an example user and a ground truth format for said user.

6. A method as in claim 5 wherein the ground truth format comprises a format that would lead the example user to correctly determine whether to initiate an example medical intervention.

7. A method as in claim 4 wherein the model comprises a reinforcement learning model and wherein the method further comprises providing feedback to the reinforcement model, the feedback indicating whether the user correctly initiated the medical procedure when the risk score was displayed in the determined format.

8. A method as in claim 4 wherein the model comprises a fuzzy neural network or wherein the model uses differential inductive logic to determine the format.

9. A method as in any one of the preceding claims further comprising determining a cost effectiveness of performing the medical intervention and;
wherein the step of determining a format in which to display the risk score to the user is further based on the determined cost effectiveness.

10. A method as in claim 9 wherein the step of determining a format in which to display the risk score to the user comprises:
selecting a format that is more likely to result in the user initiating the medical intervention if the medical intervention is determined to be cost effective compared to if the medical intervention is determined to be less cost effective.

11. A method as in claim 9 wherein the step of determining a format in which to display the risk score to the user comprises:
selecting a format that is more likely to result in the user initiating the medical intervention if a cost associated with not performing the medical intervention is higher than a cost associated with performing the medical intervention.

12. A method as in any one of the preceding claims wherein the step of determining a format comprises:
selecting a format that is less likely to result in the user initiating the medical intervention if previous risk scores displayed to the user have resulted in the user initiating unnecessary medical interventions, compared to if previous risk scores displayed to the user have resulted in the user initiating necessary medical interventions.

13. An apparatus for displaying to a user a risk score associated with a risk of a patient requiring a medical intervention, the apparatus comprising:
a memory comprising instruction data representing a set of instructions; and
a processor configured to communicate with the memory and to execute the set of instructions, wherein the set of instructions, when executed by the processor, cause the processor to:
obtain the risk score for the patient;
determine a format in which to display the risk score to the user based on a numerical literacy of the user; and
send an instruction to a user display to instruct the user display to display the risk score to the user in the determined format.

14. An apparatus as in claim 13 wherein the apparatus comprises a telehealth services apparatus.

15. A computer program product comprising a computer readable medium, the computer readable medium having computer readable code embodied therein, the computer readable code being configured such that, on execution by a suitable computer or processor, the computer or processor is caused to perform the method as claimed in any one of claims 1 to 12.
